(19) Europäisches Patentamt
European Patent Office
Office européen des brevets

(11)  EP 2 948 059 B1

# (12) EUROPEAN PATENT SPECIFICATION

(45) Date of publication and mention
of the grant of the patent:
**15.02.2017  Bulletin 2017/07**

(21) Application number: **14729899.6**

(22) Date of filing: **13.06.2014**

(51) Int Cl.:
*A61B 5/00* *(2006.01)*       *A61B 5/1455* *(2006.01)*
*A61B 5/145* *(2006.01)*     *A61B 5/01* *(2006.01)*
*A61M 16/00* *(2006.01)*   *A61M 16/06* *(2006.01)*
*A61M 16/10* *(2006.01)*   *A61M 16/12* *(2006.01)*

(86) International application number:
**PCT/EP2014/062324**

(87) International publication number:
**WO 2014/198868 (18.12.2014 Gazette 2014/51)**

## (54) DEVICE AND METHOD FOR DETERMINING A PARTIAL CARBON DIOXIDE PRESSURE IN A SUBJECT OF INTEREST

VORRICHTUNG UND VERFAHREN ZUR BESTIMMUNG EINES PARTIELLEN KOHLENDIOXIDDRUCKS IN EINER PERSON VON INTERESSE

DISPOSITIF ET PROCÉDÉ POUR DÉTERMINER UNE PRESSION PARTIELLE DE DIOXYDE DE CARBONE CHEZ UN SUJET D'INTÉRÊT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.06.2013  EP 13171767**

(43) Date of publication of application:
**02.12.2015  Bulletin 2015/49**

(73) Proprietor: **Koninklijke Philips N.V.**
**5656 AE Eindhoven (NL)**

(72) Inventor: **KAHLMAN, Josephus Arnoldus Henricus Maria**
**NL-5656 AE Eindhoven (NL)**

(74) Representative: **Gravendeel, Cornelis**
**Philips Intellectual Property & Standards**
**High Tech Campus 5**
**5656 AE Eindhoven (NL)**

(56) References cited:
**WO-A2-98/03847     GB-A- 2 485 558**

**Description**

FIELD OF THE INVENTION

The present invention relates to a device and a method for determining a partial carbon dioxide pressure in blood in a circulatory system of a subject of interest such as patient. The present invention further relates to a system for ventilating a patient making use of new approaches in determining a partial carbon dioxide pressure in blood in a circulatory system of a subject of interest. More generally, the present invention relates to the detection of vital parameters or, even more generally, vital signs information making use of non-obtrusive monitoring which may even comprise so-called remote monitoring approaches. More specifically, the present invention may relate to image processing systems and methods in a field of medical imaging which can be applied in the field of remote monitoring, such as remote photoplethysmographic monitoring, remote oxygen saturation detection and related applications.

The present invention further relates to a corresponding computer program.

BACKGROUND OF THE INVENTION

GB 2 485 558 A relates to a device and a method for an invasive blood analysis apparatus and method, wherein blood is provided from a patient to an oxygenator, probed and then provided back to the patient. Blood properties are directly measured from the blood provided to or from the oxygenator. Consequently, probing the patient's blood is particularly obtrusive.

WO 98/03847 A2 discloses a method and device for non-invasively determining blood parameters. The device may comprise a temperature inducement generator for inducing temperature changes in a patient's blood and a temperature measurement means for measuring the temperature of the blood. Furthermore, a controller for computing various blood parameters based on induced temperature levels of the blood is provided.

WO 2012/077065 A1 discloses a method and an apparatus for determining a partial carbon dioxide pressure in arterial blood of a subject, the method comprising the steps of:

- causing a modulation of at least one parameter of arterial blood in a subject;
- deriving oxygen saturation of the arterial blood of said subject during modulation of said at least one parameter; and
- determining the partial carbon dioxide pressure of said subject from said derived oxygen saturation.

The document further discloses several refinements of the method and the apparatus. The document particularly addresses monitoring the partial arterial oxygen pressure ($PaO_2$) of a patient's blood by measuring the arterial oxygen saturation ($SaO_2$) and then using the oxygen dissociation curve (ODC) to derive the partial arterial oxygen pressure ($PaO_2$) from the measured arterial oxygen saturation ($SaO_2$). With respect to the detection of the partial pressure of carbon dioxide ($PaCO_2$), the document addresses so-called capnography. Capnography is a known technique for monitoring the inhaled and exhaled concentration of partial pressure of $CO_2$, and thus indirectly monitoring the $CO_2$ partial pressure in the arterial blood.

Another known technique for monitoring $PaCO_2$ is transcutaneous $CO_2$ monitoring. Typically, transcutaneous $CO_2$ monitoring makes use of an electrochemical or chemo-optical sensor which is attached to a patient's skin. Furthermore, the skin tissue is heated to promote arterialization, which is needed to relate the measured transcutaneous $CO_2$ pressure ($PtcCO_2$) with the arterial $CO_2$ pressure ($PaCO_2$). As known in the art, the $CO_2$ pressure of the tissue may be different from the actual arterial $CO_2$ pressure which basically decreases the accuracy of this approach. Transcutaneous $CO_2$ sensors typically require some heating to increase of temperature which increases the level of accuracy of the $PaCO_2$ determination. Consequently, temperature correction is required.

Transcutaneous sensors typically require re-calibration and re-positioning due to thermal influences, such as skin burn, to the patient's skin caused by active temperature management. Therefore transcutaneous monitoring is often considered unpleasant. Particularly, transcutaneous monitoring is not well-suited for long time monitoring.

For many healthcare applications, blood measurement and monitoring is crucial for assessing a patient's (or, more generally, a subject's) respiratory condition. This basically may apply to intensive-care medicine, inpatient treatment, and also to outpatient treatment. Especially for ventilated patients suffering from various pulmonary diseases, arterial blood measurements may be considered as a widely applied standard for spot-check measurements.

As mentioned above, non-invasive monitoring approaches allow for monitoring parameters such as partial gas pressures ($PaO_2$, $PaCO_2$) and oxygen saturation ($SpO_2$) in blood by physically attaching sensors to the patient's body. Still, however, these known techniques can still be considered as being obtrusive since sensor elements have to be fixed to the patient's body.

While the required preparation, installation and surveillance can be provided and ensured in hospitals for inpatient treatment, especially outpatient treatment of patients staying at home may not be capable of providing the required monitoring performance in terms of preparation and installation efforts and monitoring accuracy in any case.

Home-respiratory care is an appropriate measure for patients suffering from diseases such as chronic obstructive pulmonary disease (COPD) or neuro-muscular diseases which are typically ventilated by means of non-invasive ventilation (NIV) at home. Typically, these patients initially stay at the hospital so as to assess and optimize ventilating settings and for monitoring arterial blood gas waves. Depending on the course of the disease, the severity and the patient's stability, the patients may have to return from time to the hospital for additional checks and set-ups. In the alternative, qualified medical staff, such as a respiratory nurse, may visit the patient at home so as to control the ventilating arrangement and parameters. Also during these visits, blood gas monitoring arrangement can be arranged and applied to the patient. For instance, blood gas monitoring can be performed over night which may be coupled with data acquisition with respect to ventilation and respiratory data, whereas the accumulated data can be provided for subsequent analysis by the qualified medical staff.

As known in the art, partial carbon dioxide monitoring can be performed non-invasively by capnography. Capnography can be considered as an appropriate approach for intubated patients having a considerably healthy lung. In this connection, capnography aims at the determination of an end tidal carbon dioxide ($EtCO_2$) value which may serve as a proper indication of an arterial carbon dioxide value. However, in some applications, for instance with patients suffering from severe respiratory diseases and/or when non-invasive ventilation is applied where air may leak through gaps between a ventilation mask and the patient's face, capnography may not provide sufficient reliability. Typically, for a stationary treatment in hospitals, capnography is combined with obtrusive invasive arterial blood sampling so as to obtain on a one-hand side accurate values from time to time while being able for permanently monitoring and analyzing trend values that are less accurate than values based on blood sampling but that may still be diagnostically conclusive.

According to another approach, transcutaneous carbon dioxide monitoring is not disturbed or affected by air-leakages and severe respiratory defects in the patient. However, transcutaneous $CO_2$ monitoring still requires qualified medical staff for installing the measurement equipment and initiating and observing accurate and precise measurements. Frequently, transcutaneous carbon dioxide monitoring has been reported to the susceptible to skin property variations. Consequently, care should be taken when performing such measurements. Otherwise, inaccurate values may not be unlikely. Currently, carbon dioxide blood gas monitoring is not commonly applied to outbound patients staying at home so a high relevance for patients receiving non-invasive ventilation is acknowledged.

By way of example, a commonly known transcutaneous carbon dioxide sensor may comprise of a thermostatically controlled heater element configured for increasing blood perfusion and gas-permeability of the patient's skin, a fluid layer provided between the skin and a sensor membrane; a gas-permeable membrane covering a sensor, a sensor comprising an electrochemical pH sensor and a reference electrode, and a processing unit configured for applying a compensation algorithm to compensate for temperature effects and skin metabolism.

Temperature effects may arise since a difference between the temperature present at the sensor and an (assumed) arterial blood temperature are typically different and have to be taken into account when deriving the desired "transcutaneous" carbon dioxide value from the measured "cutaneous" partial carbon dioxide pressure.

Also in case of transcutaneous measurements temperature effects may evolve from a slight heating process which is applied to skin tissue adjacent to a sensor surface of a contact sensor. Sensor heating may be required for enhance skin arterialization which is considered to be important for transcutaneous blood gas measurements so as to be able to derive transcutaneous values reflecting the arterial blood gas levels. For instance, in some currently available transcutaneous measurement systems an appropriate minimum sensor temperature for arterialization may be about 42 °C (Celsius) which may imply a heating power input of about 500 mW (milliwatt) at maximum so as to compensate for cooling effects caused by the blood flow.

A further approach to partial carbon dioxide pressure measurements can be based on photoplethysmographic measurement methods, such as pulse oximetry. Plethysmography generally refers to the measurement of volume changes of an organ or a body part and in particular to the detection of volume changes due to a cardio-vascular pulse wave traveling through the body of a subject with every heart beat.

Photoplethysmography (PPG) is an optical measurement technique that evaluates a time-variant change of light reflectance or transmission of an area or volume of interest. PPG is based on the principle that blood absorbs light more than surrounding tissue, so variations in blood volume with every heart beat affect transmission or reflectance correspondingly. Besides information about the heart rate, a PPG waveform can comprise information attributable to further physiological phenomena such as the respiration. By evaluating the transmissivity and/or reflectivity at different wavelengths (typically red and infrared), the blood oxygen saturation can be determined.

Conventional pulse oximeters for measuring the heart rate and the (arterial) blood oxygen saturation of a subject are attached to the skin of the subject, for instance to a finger tip, earlobe or forehead. Therefore, they are referred to as 'contact' PPG devices. A typical pulse oximeter comprises a red LED and an infrared LED as light sources and one photodiode for detecting light that has been transmitted through patient tissue. Commercially available pulse oximeters quickly switch between measurements at a red and an infrared wavelength and thereby measure the transmissivity of the same area or volume of tissue at two different wavelengths. This is referred to as time-division-multiplexing. The transmissivity over time at each wavelength gives the PPG waveforms for red and infrared wavelengths. Although contact PPG is regarded as a basically non-invasive technique, contact PPG measurement is often experienced as being unpleasant, since the pulse oximeter is directly attached to the subject and any cables limit the freedom to move.

Recently, non-contact, remote PPG devices for unobtrusive measurements have been introduced. Remote PPG utilizes light sources or, in general radiation sources, disposed remotely from the subject of interest. Similarly, also a detector, e.g., a camera or a photo detector, can be disposed remotely from the subject of interest. Therefore, remote photoplethysmographic systems and devices are considered unobtrusive and well suited for medical as well as non-medical everyday applications.

For instance, the partial $CO_2$ pressure in arterial blood of a subject, for example, a human patient, can be determined by measuring and/or deriving oxygen saturation of the arterial blood of the subject at intervals as the concentration of the gas components supplied to the patient via a non-invasive ventilator, for example, are modulated. This may be achieved by synchronizing the derivation of the oxygen saturation with the modulation (e.g., temperature modulation) cycle of the parameter modulation of arterial blood in the subject. The partial carbon dioxide pressure may then be determined from the derived oxygen saturation. This may be achieved by deriving the pH of the arterial blood of the patient from the changes in the oxygen saturation of the arterial blood due to the modulation and, using the Oxygen Dissociation Curve (ODC), deriving the pH value from these changes.

The oxygen saturation of blood is the fraction oxyhemoglobin with respect to the total amount of hemoglobin, i.e. oxyhemoglobin plus deoxyhemoglobin as a function of the partial oxygen pressure. The OHDC describes the relation between the partial oxygen pressure (pO2) and the oxygen saturation (Oxygen Hemoglobin Dissociation Curve).

As basically shown in WO 2012/077065 A1, the oxygen saturation of the arterial blood of said subject may be derived by measuring the oxygen saturation of the arterial blood of the subject using simple and robust probes such as a pulse oximeter. This measuring may be synchronized with the (active) modulation cycle of the supplied gas concentrations, for instance with active temperature modulation. Therefore, the resulting modulation of the partial pressures may be small (+/- 1 kPa).This can be achieved by measuring the $SpO_2$ modulation in synchronous with the modulation cycle. An additional advantage of this measuring scheme is that variations in the actual $SpO_2$ value due to physiological processes do not impair the modulation measurement, since they are essentially decoupled.

The arterial blood of the subject may be modulated at a rate such that the $PaO_2$ is also modulated. Oxygen saturation readings may be taken at different $PaO_2$ levels and, using the oxygen dissociation curve, the pH can be determined and eventually, based on the pH, the partial carbon dioxide pressure can be determined. This has proved to provide a measure of the partial carbon dioxide pressure at about 0.2kPa accuracy. The partial carbon dioxide pressure may be determined using a simplified and standardized form of the Henderson-Hasselbalch equation as disclosed by Clinical and Laboratory Standard Institute (CLSI) in *Guidelines for Blood Gas and pH Analysis and Related Measurements*.

Alternatively, approximations for certain working points in the oxygen dissociation curve can be obtained, e.g.:

$$pH \approx pKa - \beta\, PaCO_2 , \qquad\qquad\qquad (1)$$

wherein pH is the pH value of the arterial blood of said subject, pKa is an ionization constant of the arterial blood of the subject, pKa being preferably within the range of 7.5 to 8.0, $\beta$ is a personal coefficient of the subject, $\beta$ being preferably within the range of 0.04 to 0.08 and $PaCO_2$ is the arterial partial carbon dioxide pressure. In a typical patient, pKa is about 7.7 and $\beta$ is about 0.06.

On the basis of oxygen saturation values determined via photoplethysmography, a partial oxygen pressure can be derived via a so-called oxygen dissociation curve (OHDC). Furthermore, as shown in WO 2012/077065 A1, oxygen saturation detection via photoplethysmography ($SpO_2$) can be extended in a favorable manner so as to finally derive partial carbon dioxide pressure values ($pCO_2$) therefrom. To this end, the document proposes to actively modulate the blood of the observed patient at a side of measurement of the oxygen saturation.

Blood gas monitoring via blood sampling is considered to be particularly obtrusive and is therefore found unpleasant by the monitored subjects. Also for transcutaneous blood gas measurements making use of sensors that have to be attached to a subject's skin still a lot of preparation, attachment and calibration work is necessary which is also found to be obtrusive and unpleasant. Particularly, temperature management and/or temperature modulation with respect to a blood flow in the subject to be monitored requires costly devices and considerable preparation and surveillance operations. Consequently, the application of blood gas measurement and monitoring is limited. This applies in particular to outpatient treatment of subjects staying at home. Still, however, from a medical point of view, it would be beneficial to allow for blood gas measurement and monitoring also for patients staying at home.

## SUMMARY OF THE INVENTION

It is an object of the present invention to provide an improved device and method for unobtrusively and economically determining a blood gas partial pressure in blood in a circulatory system of a subject of interest. It would be further advantageous to provide a system for ventilating a patient making use of such a device and method. Advantageously, the device and method of the present disclosure provide further refinements in processing detected signaled so as to allow for improvements in automatically monitoring and detecting blood gas fractions and/or pressure values. Furthermore, it would be advantageous to present a device and a related method that allow for unobtrusive monitoring, and that are particularly well-suited for long-term monitoring.

In a first aspect of the present invention a device for unobtrusively determining a blood gas partial pressure in blood in a circulatory system of a subject of interest is presented, the device comprising:

- a non-obtrusive temperature detector for detecting temperature-related measurement values indicative of present intrinsic natural blood temperature level differences;
- a non-obtrusive oxygen saturation sensor for deriving oxygen saturation measurements of the blood of said subject under consideration of said temperature-related measurement values; and
- a blood gas pressure processor for determining a blood gas partial pressure of the monitored subject from said derived oxygen saturation measurements under consideration of said temperature-related measurement values that are indicative of the present blood temperature level differences of said subject.

The present invention is based on the insight that intrinsic natural blood temperature differences and/or changes present in a subject to be monitored (or: a patient) may be utilized to determine oxygen saturation ($SpO_2$) and its actual dependency on blood temperature. Thus, mere $SpO_2$-detection can be extended to blood gas partial pressure measurement since, for instance, the relation among the $SpO_2$ values and the corresponding temperature values can be considered in connection with a formal correlation such as the hemoglobin oxygen dissociation curve based on which the desired blood gas partial pressure can be derived. For instance, the determination of partial carbon dioxide pressure in the subject's arterial blood can be addressed. Furthermore, also the partial oxygen pressure can be an object of a measurement. Needless to say, also measurements deduced therefrom can be derived. In other words, when tackling a determination of the partial carbon dioxide pressure in the subject's blood, pairs of values comprising oxygen saturation-representative values and temperature-representative values can be detected at basically the same time which allows for deducing the partial carbon dioxide pressure ($PaCO_2$) from the oxygen hemoglobin dissociation curve (OHDC) under consideration of another formal relation. In this connection, reference is made to WO 2012/077065 A1 which is presenting an example making use of a simplified and standardized form the Henderson-Hasselbalch which is disclosed by Clinical and Laboratory Standard Institute (CLSI) in *Guidelines for blood gas and pH analysis and related measurements.* Furthermore, WO 98/03847 A2 is referred too in this regard. The document discloses a device for non-invasive determination of blood parameters. Also this document addresses the computation of carbon dioxide parameters in a patient's blood. Furthermore, the use of the Henderson-Hasselbalch equation is described in this document.

However, in contrast to the prior art, the device of the present disclosure is further configured for determining and detecting temperature changes rather than for inducing them. Consequently, active temperature management is replaced by "passive" temperature measurement which allows for a simplified measurement and processing equipment. In this way, a fairly unobtrusive blood gas pressure measurement is achieved. This applies in particular when the temperature detector and the oxygen saturation sensor are arranged at non-obtrusive sensors. Preferably, at least one of the temperature detector and the oxygen saturation sensor is configured as an optical sensor. More preferably, at least one of the temperature detector and the oxygen saturation sensor is arranged at a remote optical sensor allowing for non-contact measurements.

As indicated above, non-contact photoplethysmographic approaches for oxygen saturation detection have been introduced recently. In this connection reference is made to Verkruysse et al., "Remote plethysmographic imaging using ambient light", Optics Express, 16(26), 22 December 2008, pp. 21434-21445 demonstrating that photoplethysmographic signals can be measured remotely using ambient light and a conventional consumer level video camera.

Camera based, contactless physiological measurements require enough light of appropriate wavelengths to extract the desired vital sign information. This can be achieved by ensuring, that the ambient illumination is set up accordingly. During sleep time, however, it can be a big discomfort for patients or other monitored subjects, if the light has to be turned on during a measurement. Furthermore, it is important that the optical spectrum of the illumination meets the requirements. For example, it is important for measuring the oxygen saturation of blood to have light of defined wavelengths, typically red and infrared.

Wieringa, et al., "Contactless Multiple Wavelength Photoplethysmographic Imaging: A First Step Toward "SpO2 Camera" Technology," Ann. Biomed. Eng. 33, 1034-1041 (2005), discloses a remote PPG system for contactless imaging of arterial oxygen saturation in tissue based upon the measurement of plethysmographic signals at different wavelengths.

The system comprises a monochrome CMOS-camera and a light source with LEDs of three different wavelengths. The camera sequentially acquires three movies of the subject at the three different wavelengths. The pulse rate can be determined from a sequence at a single wavelength, whereas at least two movies at different wavelengths are required for determining the oxygen saturation. According to Wieringa, et al., the measurements are performed in a dark room, using only one wavelength at a time.

Contact pulse oximeters typically transmit red (R) and infrared (IR) (or, more precisely, in some cases near infrared) light through a vascular tissue of the subject of interest. The respective light portions (R/IR) can be transmitted and detected in an alternating (fast-switching) manner. Given that the respective spectral portions are differently absorbed by oxygenated hemoglobin ($HbO_2$) and reduced hemoglobin (Hb), blood oxygen saturation eventually can be processed. An oxygen saturation ($SO_2$) estimation algorithm can make use of a ratio of the signals related to the red and the infrared portion. Furthermore, the algorithm can consider a non-pulsatile signal component. Typically, the PPG signal comprises a DC component and a relatively small pulsatile AC component. Furthermore, $SO_2$ estimation generally involves an empirically derived calibration factor applied to the processed values.

Typically, the calibration factor (or, calibration curve) is determined upon reference measurements involving invasive blood oxygen saturation measurements. A calibration factor is required since a PPG device basically detects a ratio of (spectral) signal portions which has to be transferred into a blood oxygen saturation value which typically involves a ratio of $HbO_2$ and Hb. For instance, but not intended to limit the present disclosure, blood oxygen saturation estimation can be based on the following general equation:

$$ SO_2 = \frac{HbO_2}{HbO_2 + Hb}, \tag{2} $$

whereas PPG devices merely detect $HbO_2$ and Hb from the spectral response at at least two wavelengths in a mediate way.

Generally, the measured intensity curve 28, 29 as a characteristic signal is considered to contain a considerably constant (DC) portion and an alternating (AC) portion superimposing the DC portion. Applying signal processing measures, the AC portion can be extracted and, furthermore, compensated for disturbances. For instance, the AC portion of the characteristic signal can comprise a dominant frequency which can be highly indicative of the subject's vascular activity, in particular the heart beat. Still, the characteristic signal, in particular the AC portion, can be indicative of further vital parameters. In this connection, the detection of arterial blood oxygen saturation is an important field of application.

As indicated above, basically, arterial blood oxygen saturation-representative values can be computed taking into account the behavior of the AC portion of the characteristic signal at distinct spectral portions thereof. In other words, a degree of arterial blood oxygen saturation can be reflected in different radiation absorbance at blood vessels. Furthermore, one can make use of the fact that the difference in absorbance due to the grade of oxygenation also varies significantly across different spectral portions. Moreover, also the DC portion of the signal can be utilized for blood oxygen saturation detection. Typically, the DC component represents the overall light absorption of the tissue, venous blood, and non-pulsatile arterial blood. By contrast, the AC component may represent the pulsatile arterial blood's absorption. Consequently, the determination of arterial blood oxygen saturation ($SaO_2$) can be expressed as:

$$ SaO_2 = C \cdot \frac{(AC/DC)_{red}}{(AC/DC)_{infrared}}, \tag{3} $$

where C is a calibration parameter. C may stand for a large variety of calibration parameters applicable to the AC/DC relationship and should therefore not be interpreted in the strict algebraic sense of equation (3). C may, for example, represent a fixed constant value, a set of fixed constants or an adjustable calibration parameter. By way of example, another exemplary $SaO_2$ derivation model can be expressed as:

$$ SaO_2 = C_1 + C_2 \cdot \frac{(AC/DC)_{red}}{(AC/DC)_{infrared}}, \tag{4} $$

where $C_1$ and $C_2$ can be considered calibration parameters of a linear approximation. In an exemplary embodiment, the signal calibration parameter determination can be directed to adjust or adapt the parameter $C_1$. Still, in the alternative, $SaO_2$ derivation may also be based on value tables deposited in (or accessible by) an analysis unit. The value tables (or: data bases) may provide for a discrete representation of the relationship between detected PPG signals und the desired calibration parameter. Also in that case an adaptable calibration parameter may be applied to improve the accuracy of the vital parameter determination.

It should be understood that the equations (3) and (4) are primarily presented for illustrative purposes. They should not be construed as limiting the scope of the present disclosure. In practice, the skilled person may determine and establish further appropriate $SaO_2$ derivation models. Alternative wavelength combinations, for example green and red, can be used depending on the substance to be detected. While the measurement of $SaO_2$ has been described in detail, this is to be understood as an example for the general concept of measuring the concentration of a substance in blood and/or tissue.

The device for determining blood gas partial pressure, particularly for determining partial carbon dioxide pressure in blood, can make use of such principles for $SpO_2$ detection. Furthermore, alternatively, or in addition, a remote temperature detector such as a thermal camera, can be utilized for remotely sensing a temperature at or in proximity to a measurement side for the oxygen saturation measurement. Needless to say, the temperature detector can be configured for detecting temperature-related measurement values since basically a skin temperature (surface temperature) rather than a blood temperature (beneath the skin surface) can be detected by the temperature detector. However, based on the temperature-related measurement, blood temperature levels can be calculated and derived.

In this way, an appropriate basis for the determination of the blood gas partial pressure such as the partial carbon dioxide pressure can be established since appropriate pairs of oxygen saturation values and related temperature-related values can be obtained.

According to another aspect of the present disclosure, the blood gas partial pressure is a partial carbon dioxide pressure, wherein the blood gas pressure processor is arranged as a partial carbon dioxide pressure processor, and wherein the blood gas pressure processor is further configured for determining pH-representative values attributable to present blood pH-values of the subject.

In this way the device is arranged to determine the partial carbon dioxide pressure from said derived oxygen saturation measurements by deriving the pH-values of the arterial blood of the subject under consideration of an oxygen dissociation curve. Furthermore, the Henderson-Hasselbalch equation can be taken into account when deriving the partial carbon dioxide pressure.

Since oxygen saturation detection can be performed along with temperature detection, it may be assumed that changes in said derived oxygen saturation measurements are basically caused by temperature changes detected by the temperature detector. Given this relationship, pH-values can be derived from the oxygen dissociation curve (ODC). Consequently, under consideration of the Henderson-Hasselbalch equation, the blood gas partial pressure, namely the partial carbon dioxide pressure, can be calculated.

According to another aspect the temperature detector is further configured for monitoring at least two measurement zones at the subject's skin, wherein the at least two measurement zones exhibit different blood temperature levels.

Consequently, also the oxygen saturation sensor should be configured accordingly, that is, preferably temperature detection and oxygen saturation detection is performed at basically the same measurement site or at least a basically adjacent measurement sites. In this way, a close connection between the oxygen saturation values and the respective temperature values can be assured. It is emphasized in this connection that slightest temperature differences can be utilized for detecting and the deriving the desired signals. Consequently, there is no further need for modulating a blood temperature level by actively heating or cooling a measurement site at the subject's skin and performing active temperature measurement.

It is further preferred in this connection if the temperature detector is configured for detecting temperature values at a plurality of measurement spots. If, for instance, the temperature detector is arranged as an optical detector, the temperature of a considerably large surface portion of the subject of interest can be monitored. In this way, the blood gas pressure processor can be further configured for performing a temperature interpolation for interpolating an actual temperature at a present oxygen saturation measurement site on the basis of a plurality of temperature measurement sites surrounding the oxygen saturation measurement site. In this way, temperature effects at the very oxygen saturation measurement spot cannot affect the overall blood gas partial pressure determination accuracy.

The blood gas pressure processor may be further configured for selecting measurement spots and/or measurement locations on the basis of various optimization requirements. For instance, a measurement site can be selected which ensures minimal partial carbon dioxide pressure in a present measurement. In other words, a plurality of measurement spots or sites can be monitored while, on the basis of an optimization algorithm, only those spots or sites are selected for further processing that may achieve a high accuracy.

According to another aspect of the device, the temperature detector can be arranged as an optical detector comprising at least one sensory element for sensing electromagnetic radiation indicative of actual temperature values.

This embodiment can be further developed in that the temperature detector is arranged as a thermal imaging sensor, particularly as a thermal imaging camera.

Basically, a thermal imaging sensor can be arranged as a remote sensor, for instance as a sensor comprising at least one photodiode which is capable of sensing infrared radiation. Such a thermal imaging sensor could be configured for being attached to the subject's skin. However, in the alternative, a thermal imaging sensor comprising at least one diode for sensing electromagnetic radiation in the infrared wavelength range could be arranged as a remote non-contact sensor. If the temperature detector is arranged as a thermal imaging camera, a plurality of sensor elements can be provided, for instance an array for sensory elements. Such an array could be arranged, for instance, as a CMOS-sensor (complementary metal-oxide-semiconductor sensor), as a CCD-sensor (charge-coupled device sensor), and such like. Preferably, the sensor arrays are configured as sensor panels. It is further preferred that these panels are operable so as to detect incident radiation in the infrared spectrum.

According to another embodiment of the device, the temperature detector further comprises at least one optical temperature indicator attachable to the subject's skin, wherein the at least one optical temperature indicator is capable of exhibiting a property change in response to a change in temperature. By way of example, the at least one optical temperature indicator can make use of so-called thermochromatic substances and materials. In this way, there is no need of a temperature detector being capable of "directly" measuring temperature at the subject of interest. For instance, making use of image processing, a detected property change of the at least one optical temperature indicator (such as a color change, can be detected and analyzed so as to derive an underlying temperature change). Given that also the oxygen saturation measurement can be performed using imaging devices, such as a video camera, basically the same imaging device can be used for detecting oxygen saturation indicative values and temperature indicative values. Video data can be captured and analyzed so as to extract temperature-related information and a skin representation including a representation of minute changes in skin properties which allow for a detection of oxygen saturation values.

This embodiment can be even further developed in that the at least one optical temperature indicator is arranged as an at least partially transparent temperature indicator. In this way, temperature detection and oxygen saturation detection can be performed at basically the same measurement site, or, at respective measurement sites that are adjacent to each other.

According to another aspect of the device, the oxygen saturation sensor is arranged as an optical sensor being capable of sensing electromagnetic radiation at at least two distinct wavelength portions indicative of blood perfusion in a subject's tissue. To this end, the oxygen saturation sensor may be provided with at least one photodiode that is coupled with a light source that is capable of selectively emitting electromagnetic radiation at at least two wavelength portions. Constantly, switching or alternating the light sources and correspondingly synchronizing the at least one photodiode may allow for an alternating detection of electromagnetic radiation at the at least two distinct wavelength portions which can be processed and analyzed so as to eventually derive oxygen saturation values. In the alternative, at least two photodiodes may be utilized each of which is assigned to a distinct wavelength portion.

According to yet another aspect of the device, the oxygen saturation sensor is arranged as an image sensor, particularly as an imaging camera capable of sensing electromagnetic radiation in at least one particular wavelength range. By way of example, the imaging camera can be capable of sensing visible radiation. It could be further advantageous, if the imaging camera is having an extended responsivity allowing for sensing electromagnetic radiation also in the infrared wavelength range. In this way, visible radiation, e.g., a skin representation, along with infrared radiation, e.g., temperature-indicative skin representation, can be captured. As indicated above, in some embodiments, a single imaging sensor, such as an imaging camera can be utilized for temperature detection and for oxygen saturation measurement detection.

It is particularly preferred if temperature detection and oxygen saturation measurement are performed on a non-obtrusive remote basis. This may imply that no detector, sensor or sensory element is attached to the subject's skin. In this way, a fairly unobtrusive measurement can be achieved. Needless to say, also such an embodiment making use of a remote imaging camera can be combined with at least one optical temperature indicator that is attached to the subject's skin.

Still, however, in some embodiments the temperature detector can be arranged as a contact sensor that is attachable to the subject's skin. In order to detect different temperature levels, the temperature detector may comprise a plurality of temperature detector elements that may be attached to the subject' skin. Consequently, different temperature levels at a subject can be detected which may be utilized for determining the desired blood gas partial pressure.

According to yet another aspect of the device, the temperature detector and the oxygen saturation sensor are further capable of operating at sampling rates allowing for detecting inter-cycle variations of blood temperature and blood oxygen saturation. It has been observed that the local temperature of pulsating blood in blood vessels may vary during the heartbeat cycle due to inter-cycle cooling effects. Consequently, it would be beneficial to synchronize temperature measurement and oxygen saturation measurement. It has been further observed that the inter-cycle blood temperature variation is typically having an amplitude that may decrease with an increasing blood volume flow and with an increasing distance between the respective blood vessel, e.g., the artery, and the skin portion to which the measurement is applied.

Detecting the inter-cycle variations and synchronizing temperature measurement and saturation measurement can contribute in significantly reducing the number of required measurement locations and/or measurement sites. In other words, measurement of temperature and oxygen saturation of blood at different locations can be at least partially replaced by measuring the temperature and the oxygen saturation at different time stages and/or time instants within a heartbeat cycle. The observed effect can be further utilized for measuring skin perfusion and similar circulatory parameters. Consequently, the device of the present disclosure may find even wider application in patient monitoring.

It has been further observed that inter-cycle thermal fluctuation in the patient's due to the heartbeat may also be an indicator that pulsating (arterial) blood is measured. Consequently, detecting these fluctuations may be considered as an indication that arterial blood gas parameters are detected which ensures that the actual measurement is diagnostically relevant and conclusive.

For instance, sampling rates may be selected in the range of about 10 to 30 Hz or even higher. As used herein, inter-cycle variations may stand for temperature variations and oxygen saturations within a heart rate cycle and/or a respiration rate cycle. Given that considerably high sampling rates are possible, the device can be advantageously configured for time-dependent detection also within a circulatory period. In this way, a high-resolution measurement can be achieved. This may be further advantageous since in this way temperature changes within a heart rate or respiration rate period can be detected along with corresponding oxygen saturation measurement values. Consequently, theoretically a single measurement site for temperature detection and a single measurement site for oxygen saturation measurement can be sufficient for deriving the desired blood gas partial pressure in accordance with the aforementioned main aspect.

Needless to say, it is further preferred if temperature detection and oxygen saturation measurement are synchronized. For instance, the blood gas pressure processor may comprise a clock generator and/or time tracker that is capable of initiating measurements at the desired sampling rate.

In a further aspect of the present invention a system for ventilating a patient is presented, the system comprising a device according to any of the proceeding aspects and a patient ventilation interface, wherein the temperature detector is attached to said patient's ventilation interface. Given that using the aforementioned device of the present disclosure, an accurate determination of blood gas partial pressure is allowed, ventilation parameters may be adjusted so as to enable an improved patient treatment. This is particularly advantageous for patients suffering from several pulmonary diseases.

It is further advantageous in this connection that at least one of the temperature detector and the oxygen saturation sensor can be attached to a patient interface, such as a ventilation mask, which is applied to the patient during the ventilation treatment. Consequently, contact monitoring or almost-contact monitoring can be achieved which may allow for simplified detectors and/or sensors. For instance, at least one diode capable of sensing infrared radiation and/or a near infrared radiation can be attached to the patient ventilation interface for temperature detection. Furthermore, a fairly simple image sensor such as a camera or an array of photodiodes can be coupled to the patient ventilation interface for detecting and monitoring oxygen saturation. Each of the temperature detector and the oxygen saturation sensor may be configured for monitoring either a skin portion of the patient that is within the patient ventilation interface or a skin portion of the patient that is located outside the patient ventilation interface.

In yet another aspect of the present invention, a method for unobtrusively determining a blood gas partial pressure in blood in a circulatory system of a subject of interest is presented, the method comprising the following steps:

- non-obtrusively detecting temperature-related measurement values indicative of present intrinsic natural temperature level differencesof a subject's blood;
- non-obtrusively deriving oxygen saturation measurements of the blood of said subject under consideration of said temperature-related measurement values; and
- determining a blood gas partial pressure of a monitored subject from said derived oxygen saturation measurements under consideration of present blood temperature level differences of said subject.

The step of determining the blood gas partial pressure may further comprise the step of determining pH-representative values attributable to present blood pH-values of the subject, preferably comprising a derivation of the pH-representative values from an oxygen dissociation curve under consideration of changes in said derived oxygen saturation measurements of the blood attributable to detect a temperature-related measurement values of the subject's blood.

In yet another aspect of the present invention, there is provided a computer program which comprises program code means for causing a computer being part of a medical device or system to perform the steps of the method when said computer program is carried out on that computer. As used herein, the term "computer" may stand for a large variety of processing devices. In other words, also mobile devices having a considerable computing capacity can be referred too as computing device, even though they provide less processing power resources than standard "computers". Needless to say, such a "computer" can be a part of a medical device and/or system. Furthermore, the term "computer" may also refer to a distributed computing device which may involve or make use of computing capacity provided in a cloud environment. The term "computer" may also relate to medical technology devices, fitness equipment devices, and monitoring devices in general, that are capable of processing data. Preferred embodiments of the disclosure are defined in the dependent claims. It should be understood that the claimed method and the claimed computer program can have similar preferred embodiments as the claimed device and as defined in the dependent device claims.

Preferred embodiments of the invention are defined in the dependent claims. It shall be understood that the claimed method has similar and/or identical preferred embodiments as the claimed device and as defined in the dependent claims.

BRIEF DESCRIPTION OF THE DRAWINGS

These and other aspects of the invention will be apparent from and elucidated with reference to the embodiment(s) described hereinafter. In the following drawings

Fig. 1 shows an example of an Oxygen Hemoglobin Dissociation Curve (OHDC) for different pH values of arterial blood of a subject as a function of partial oxygen pressure $pO_2$;

Fig. 2 shows an example of an Oxygen Hemoglobin Dissociation Curve for different pH values of arterial blood of a subject as a function of temperature;

Fig. 3 shows an example of an Oxygen Hemoglobin Dissociation Curve for different temperature values of arterial blood of a subject as a function of partial oxygen pressure $pO_2$;

Fig. 4 shows an example of a deduced Oxygen Hemoglobin Dissociation Curve (delta OHDC) for different temperature values of arterial blood of a subject as a function of partial oxygen pressure $pO_2$ deduced from the curve shown in Fig. 3;

Fig. 5 shows a simplified schematic illustration of a system according to an embodiment of the present disclosure;

Fig. 6 shows a sample frame representing a subject of interest exhibit different skin temperature levels indicative of different blood temperature levels that may be utilized for detecting the partial oxygen pressure;

Fig. 7 shows a simplified schematic illustration of an alternative system according to an embodiment of the present disclosure;

Fig. 8 shows a simplified schematic illustration of a device for determining a blood gas partial pressure that may be implemented any of the systems illustrated in Fig. 5 and 7; and

Fig. 9 shows an illustrative block diagram representing several steps of an embodiment of a method in accordance with the present disclosure.

DETAILED DESCRIPTION OF THE INVENTION

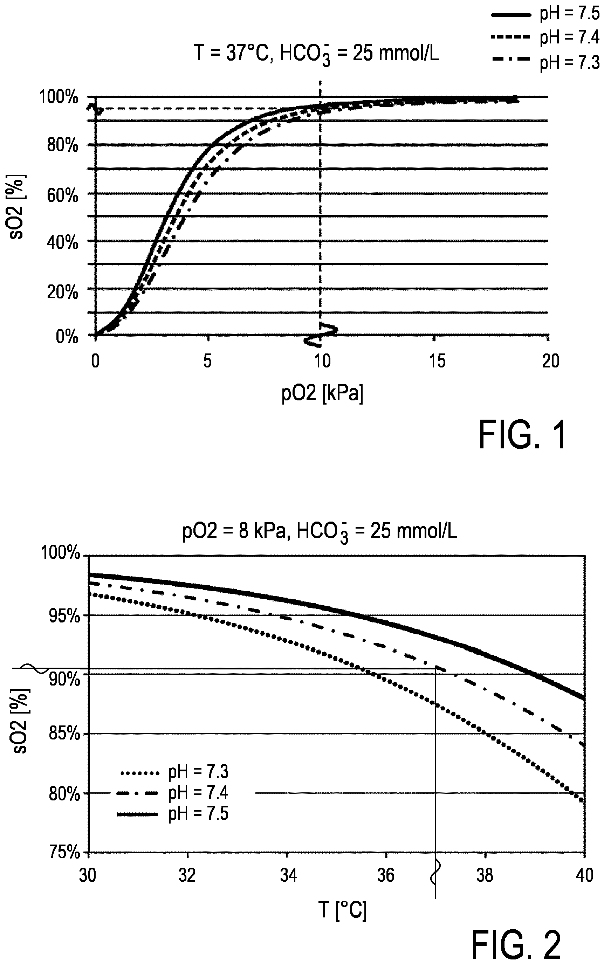

An example of an *Oxygen Dissociation Curve* is shown in Fig. 1. Fig. 1 shows the variation in the curve (relationship between the partial oxygen pressure, pO2, and the oxygen saturation, sO2) with different pH values of 7.3, 7.4 and 7.5 at a temperature T of 37°C, and bicarbonate ($HCO_3^-$) concentration of 25 mmol/L. This can be corrected for a patient's temperature $T_P$ [°C] and the concentration $HCO_3$ (bicarbonate ion) in [mmol/L] according to the Bohr Effect as follows:

$$sO_2 = \frac{X + 150X}{X^2 + 150X + 23400} 100\%, \qquad (5)$$

whereas $X = pO_2 \cdot 10^{\{0.48(pH - 7.4 - 0.0013(HCO_3 - 25)\}} \cdot 10^{\{\frac{5.49 \cdot 10^{-11} pO_2^{3.88} + 0.071}{(9.71 \cdot 10^{-9} pO_2^{3.88} + 2.3)}\}(37 - T_P)}$ in mmHg , (whereas nominal conditions are: pH= 7.4, T= 37°C, and concentration $HCO_3$ = 25mmol/L in this example).

With reference to Fig. 1, it is observed that for adult people (under 55) without any respiratory conditions, oxygen saturation should be around 97 -100% (arterial partial oxygen pressure, PaO2 > 12 kPa), whereas for patient suffering from Chronic Obstructive Pulmonary Disease (COPD) oxygen saturation generally ranges between 88-92% (7.3 < Pa02 < 8.5 kPa) since, due to their condition, they are unable to expel carbon dioxide from their lungs and the carbon dioxide is retained. Furthermore, low oxygen saturation in the range of 87-89% may be caused by sleep apnea which constricts the airway and reduces the amount of oxygen the lungs may absorb.

According to an aspect of the present disclosure, it is proposed to make use of intrinsic temperature differences in the patient's (arterial) blood, rather than merely compensating for it and/or modulating it. When modulating the temperature of the patient's blood, e.g. at the test site where a (contact) oximeter is applied, the temperature level is known beforehand. Departing from this principle, the invention proposed to detect the temperature level without influencing the temperature by defined active heating and/or cooling.

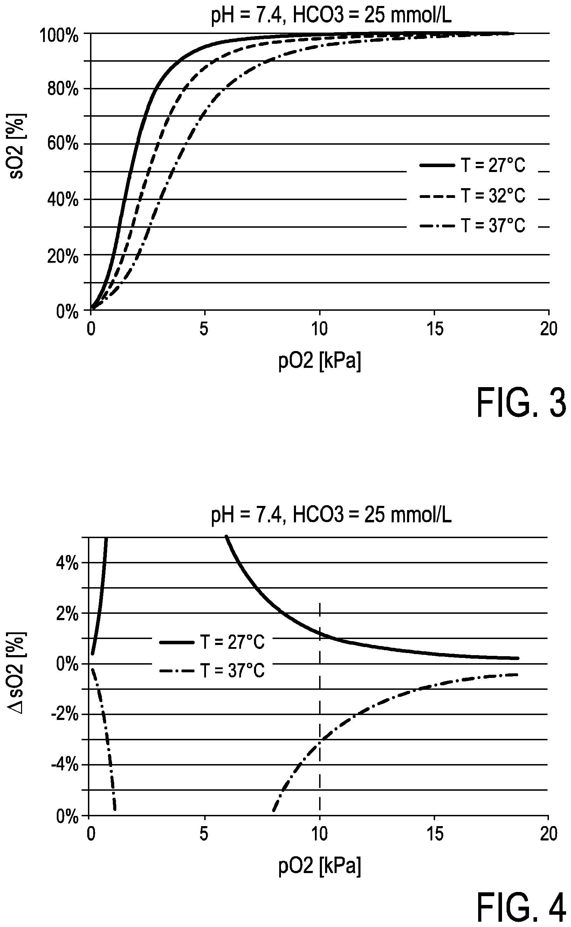

Further reference is made to Fig. 2, where several oxygen saturation curves representing different pH values are shown, illustrating oxygen saturation as a function of the local blood temperature at a constant 8 kPa partial oxygen pressure indicative for patients suffering from Chronic Obstructive Pulmonary Disease (COPD). Fig. 3 and Fig. 4 illustrate further oxygen saturation and delta oxygen saturation representative curves indicating temperature dependencies. By detecting the blood temperature (e.g., in a mediate way via skin temperature), a basically unknown parameter can be identified and, consequently, the desired partial carbon dioxide pressure can be derived from the oxygen dissociation curve (sometimes also referred to as oxygen-hemoglobin dissociation curve).

It is further emphasized that in connection with the present disclosure real unobtrusive determination of partial carbon dioxide pressure in the patient's blood can be achieved, since at least in some embodiments both oxygen saturation and blood temperature (e.g., blood temperature indicative temperature levels at the subject's skin) may be monitored and detected remotely. Since no active temperature modulation and/or temperature management is required, attachment of obtrusive contact equipment to the patient's skin can be avoided, at least to a great extent. Hereinafter, several embodiments of the device of the present disclosure are described in more detail.

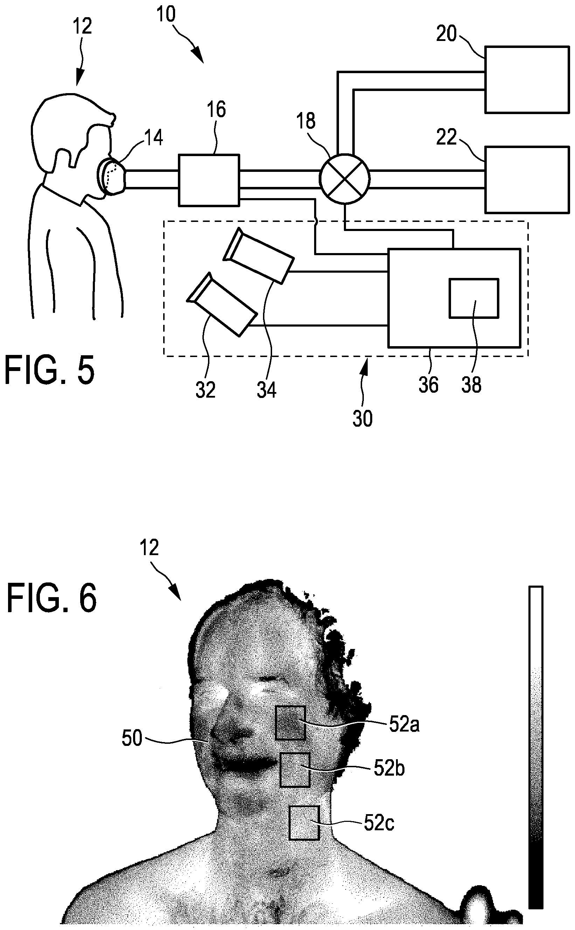

Fig. 5 illustrates an embodiment of an exemplary system 10 for ventilating a subject or patient 12 in which a device for determining a blood gas partial pressure, particularly a partial carbon dioxide pressure in the subject's 12 blood can be used. It is worth mentioning that the device disclosed in the present disclosure may be used in various fields and may find wide application. Consequently, the disclosure is not limited the ventilation systems 10 making use of such a device.

The subject 12 having non-invasive ventilation treatment is fitted with a cushioned mask 14 over its nose and mouth. More generally, the mask 14 may be referred to as patient interface, particularly as a patient interface for ventilation. The mask 14 may be supplied with a pressurized gas for the patient to breathe. For instance, the gas may comprise at least two gas components, for example, oxygen and nitrogen. These may be provided by a first gas reservoir 20 and a second gas reservoir 22. The first and second gas reservoirs 20, 22 may comprise pressurized tanks of the respective gases, or be coupled with respective conduits. The gases to be delivered to the subject are mixed by a valve 18 and fed to the mask 14 via a gas analyzer 16 for monitoring properties of the to-be-delivered gas.

Although the embodiment illustrated gas components being provide by two separate gas reservoirs 20, 22, it can be appreciated that the gases may be mixed and provided via a single reservoir such as a pressurized tank. Alternatively, the first and second gas reservoirs 20, 22 may be replaced by a compressor which pressurizes the surrounding air to be delivered to the patient or subject 12. This may include an oxygen concentrator to increase the concentration of the oxygen of the air to be delivered to the subject 12. It may also include a carbon dioxide scrubber for removing carbon dioxide from the breath exhaled from the patient to recycle the air. Many alternative arrangements for a gas source may be envisaged by persons skilled in the art.

An output of the gas analyzer 16 may be connected to a processing unit 36 provided at a device 30 for determining a blood gas partial pressure. The processing unit 36 may be coupled to the valve 18 so as to adjust the das mixture delivered to the subject 12. The processor unit 36 comprises a blood gas pressure processor 38. As to related conventional approaches and apparatuses for determining blood gas pressure, such as partial carbon dioxide pressure, WO 2012/077065 A1 is referred to.

The device 30 may further comprise several sensory elements for facilitating the determination and derivation process. For instance, at least one temperature detector 32 and at least one oxygen saturation sensor 34 may be provided. As shown in the embodiment illustrated in Fig. 5, in principle, the at least one temperature detector 32 and the at least one oxygen saturation sensor 34 may be arranged as remote contactless unobtrusive sensors. For instance, the temperature detector 32 may be arranged as an optical temperature detector, preferably as a thermal imaging sensor. The oxygen saturation sensor 34 may be arranged as an optical sensor as well. In some embodiments the sensors 32, 34 may be combined and arranged in a common housing. Given that same sensory elements are capable of sensing electromagnetic radiation in wavelength ranges that are indicative of thermal (temperature) information and of oxygen saturation information, a single type of sensor could be utilized. Monitoring oxygen saturation indicative values and blood temperature indicative values at basically the same time and basically the same location the subject's skin allows for a derivation and/or deduction of partial carbon dioxide measurements.

Fig. 6 shows a representation of a thermal image of the to-be-monitored patient or subject 12. Particularly, a neck and face portion of the subject 12 is shown. Since the image was captured with a thermal imager, such as the temperature detector 32 shown in Fig. 5, the representation contains thermal information of the subject 12. Basically, skin temperature related thermal information is provided, which is, however, also indicative of temperature of blood vessels in the subject 12. At a face portion 50 of the subject 12, several spots and/or measurements zones 52a, 52b, 52c are indicated. Consequently, different levels of temperature and oxygen saturation can be measured and utilized for detecting the desired values. Eventually, based on a correlation between changes in temperature and/or changes in oxygen saturation, blood gas pressure values can be calculated.

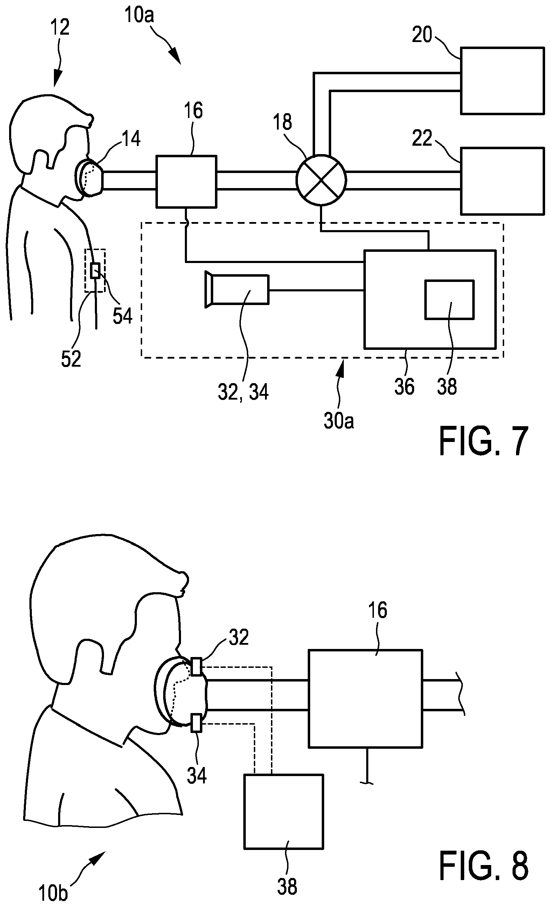

Fig. 7 shows an alternative arrangement of a device 10b for determining a blood gas partial pressure which may be coupled to a ventilating system 10a. By way of example, the device 10b comprises a single sensor body or housing in which both the (optical) temperature detector 32 and the (optical) oxygen saturation sensor 34 may be arranged. For instance, both sensors 32, 34 can make use of the same imaging device such as a CCD and/or CMOS device. A measurement zone 52 at the subject's 12 (bare) skin is indicated by a dashed line. Both sensors may observe and monitor basically the same spot or point in the measurement zone 52. For illustrating a further alternative embodiment, a temperature indicator element 54 is attached to the subject in the measurement zone. The temperature indicator element 54 may be arranged as an optical temperature indicator element 54 which may exhibit a property change when experiencing temperature changes. In this way, temperature detection and oxygen saturation detection generally may be based on the same (image) data set(s). In this embodiment, the blood gas pressure processor 38 can make use of image processing algorithms so as to derive temperature changes from any property change of the indicator element 54 detected by the temperature detector (sensor) 32. Since basically no cable connection is required for the indicator element 54, also this approach is experienced by the patient 12 as being fairly unobtrusive.

Fig. 8 illustrates that, in an alternative embodiment of a device 10b in accordance with the present disclosure, at least one of the temperature detector 32 and the oxygen saturation sensor 34 may be attached to the patient interface of mask 14. Such an arrangement comes with the benefit that the distance between the subject 12 and any sensor 32, 24 may be reduced to a great extent. Consequently, the signal-to-noise ratio can be reduced since, for instance, motion artifacts due to relative motion between the subject 12 and any sensor 32, 24 can be avoided. In this embodiment the temperature detector 32 and/or the oxygen saturation sensor 34 may be arranged as non-remote sensors. Consequently, complexity of the sensors can be reduced without impairing measurement accuracy. Needless to say, in some embodiments only one of the temperature detector 32 and the oxygen saturation sensor 34 may be provided at the mask 14 in close proximity to the patient's skin. Since a ventilating patient already has to wear a fairly obtrusive mask 14, adding at least one sensor 32, 34 to the mask 14 will most likely not be experienced by the subject 12 as even more unpleasant.

It is therefore emphasized with respect to the embodiment shown in Fig. 8 that main aspects of the present disclosure may have beneficial effects also in connection with contact-sensors. A great variety a contact sensors for temperature detection can be utilized. Also contact pulse oximeters (e.g., attachable to the subject's fingertip or earlobe) may be applicable.

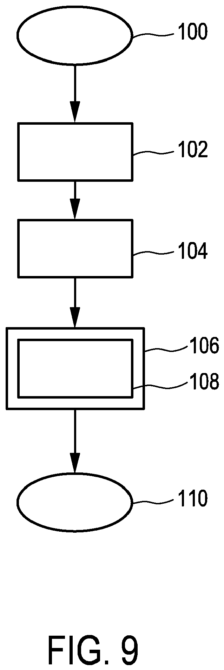

Fig. 9 schematically illustrates a method determining a blood gas partial pressure in blood in a circulatory system of a subject of interest. At a step 100, the method and a related process may be initiated. A step 102 may comprise the detection of temperature-related measurement values indicative of present temperature levels of a subject's blood. A further step may comprise the detection of oxygen saturation measurements of the blood of said subject under consideration of said temperature-related measurement values. There is no specified predefined order for the steps 102 and 104. Both steps 102, 104 may be carried out at basically the same time. In other words, pairs of values detected at basically the same time instant can be gathered. In a further step 106, a blood gas partial pressure of a monitored subject is determined from said derived oxygen saturation measurements under consideration of present blood temperature levels of said subject. The step 106 may further comprise a sub step 108 in which pH-representative values attributable to present blood pH-values of the subject are determined. Preferably, the step 108 further comprises the derivation of the pH-representative values from an oxygen dissociation curve under consideration of changes in said derived oxygen saturation measurements of the blood attributable to detected temperature-related measurement values of the subject's blood. At step 110, the method may terminate. Needless to say, the method may be used in a continuous monitoring process. Of course, also spot check monitoring is possible.

By way of example, the present invention can be applied in the field of health care, e.g. unobtrusive remote patient monitoring, general surveillances, security monitoring and so-called lifestyle environments, such as fitness equipment, or the like. Applications may include monitoring of oxygen saturation (pulse oximetry), heart rate, blood pressure, cardiac output, changes of blood perfusion, assessment of autonomic functions, and detection of peripheral vascular diseases. Needless to say, in an embodiment of the method in accordance with the invention, several of the steps described herein can be carried out in changed order, or even concurrently. Further, some of the steps could be skipped as well without departing from the scope of the invention.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1.  Device (30) for unobtrusively determining a blood gas partial pressure in blood in a circulatory system of a subject of interest (12), the device (30) comprising:

    - a non-obtrusive temperature detector (32) for detecting temperature-related measurement values indicative of present intrinsic natural blood
    temperature level differences;
    - a non-obtrusive oxygen saturation sensor (34) for deriving oxygen saturation measurements of the blood of said subject (12) under consideration of said temperature-related measurement values; and
    - a blood gas pressure processor (38) for determining a blood gas partial pressure of the monitored subject (12) from said derived oxygen saturation measurements under consideration of said temperature-related measurement values indicative of the present blood temperature level differences of said subject (12).

2.  Device (30) as claimed in claim 1, wherein the blood gas partial pressure is a partial carbon dioxide pressure, wherein the blood gas pressure processor (38) is arranged as a partial carbon dioxide pressure processor, and wherein the blood gas pressure processor (38) is further configured for determining pH-representative values attributable to present blood pH-values of the subject (12).

3.  Device (30) as claimed in claim 1, wherein the temperature detector (32) is further configured for monitoring at least two measurement zones (52a, 52b, 52c) at the subject's skin, wherein the at least two measurement zones (52a, 52b, 52c) exhibit different blood temperature levels.

4.  Device (30) as claimed in claim 1, wherein the temperature detector (32) is arranged as an optical detector comprising at least one sensory element for sensing electromagnetic radiation indicative of actual temperature values.

5.  Device (30) as claimed in claim 4, wherein the temperature detector (32) is arranged as a thermal imaging sensor, particularly as a thermal imaging camera.

6.  Device (30) as claimed in claim 4, wherein the temperature detector (32) further comprises at least one optical temperature indicator (54) attachable to the subject's skin, wherein the at least one optical temperature indicator is capable of exhibiting a property change in response to a change in temperature (54).

7.  Device (30) as claimed in claim 1, wherein the oxygen saturation sensor (34) is arranged as an optical sensor capable of sensing electromagnetic radiation at at least two distinct wavelength portions indicative of blood perfusion in a subject's tissue.

8.  Device (30) as claimed in claim 7, wherein the oxygen saturation sensor (34) is arranged as an image sensor, particularly as an imaging camera capable of sensing electromagnetic radiation in at least one particular wavelength range.

9.  Device (30) as claimed in claim 1, wherein the temperature detector (32) is arranged as a contact sensor attachable to the subject's skin.

10. Device (30) as claimed in claim 1, wherein the temperature detector (32) and the oxygen saturation sensor (34) are further capable of operating at sampling rates allowing for detecting inter-cycle variations of blood temperature and blood oxygen saturation.

11. Device (30) as claimed in claim 1, further comprising a clock generator for synchronizing the temperature detector (32) and the oxygen saturation sensor (34).

12. System for ventilating a patient, the system comprising

    - a device (30) according to any of the preceding claims; and
    - a patient ventilation interface (14);

    wherein the temperature detector (32) is preferably attached to said patient ventilation interface (14).

**13.** Method for unobtrusively determining a blood gas partial pressure in blood in a circulatory system of a subject (12) of interest, comprising the following steps:

- non-obtrusively detecting temperature-related measurement values indicative of present intrinsic natural temperature level differencesof a subject's blood;
- non-obtrusively deriving oxygen saturation measurements of the blood of said subject (12) under consideration of said temperature-related measurement values; and
- determining a blood gas partial pressure of said monitored subject (12) from said derived oxygen saturation measurements under consideration of present blood temperature level differences of said subject (12).

**14.** Method as claimed in claim 13, wherein the step of determining the blood gas partial pressure comprises:

- determining pH-representative values attributable to present blood pH-values of the subject (12), preferably comprising a derivation of the pH-representative values from an oxygen dissociation curve under consideration of changes in said derived oxygen saturation measurements of the blood attributable to detected temperature-related measurement values of the subject's blood.

**15.** Computer program comprising program code means for causing a computer being part of a medical system or device to carry out the steps of the method as claimed in claim 13 when said computer program is carried out on the computer.

**Patentansprüche**

**1.** Vorrichtung (30) zur unaufdringlichen Bestimmung eines partiellen Blutgasdrucks in Blut in einem Kreislaufsystem einer Person von Interesse (12), wobei die Vorrichtung (30) Folgendes umfasst:

- einen nicht-belästigenden Temperaturdetektor (32) zum Detektieren von temperaturbezogenen Messwerten, die die vorliegenden intrinsischen natürlichen Bluttemperaturpegeldifferenzen angeben;
- einen nicht-belästigenden Sauerstoffsättigungssensor (34) zum Ableiten von Sauerstoffsättigungsmessungen des Bluts der genannten untersuchten Person (12) unter Berücksichtigung der genannten temperaturbezogenen Messwerte; und
- einen Blutgasdruckprozessor (38) zum Ermitteln eines partiellen Blutgasdrucks der überwachten Person (12) anhand der genannten abgeleiteten Sauerstoffsättigungsmessungen unter Berücksichtigung der genannten temperaturbezogenen Messwerte, die die vorliegenden Bluttemperaturpegeldifferenzen der genannten Person (12) angeben.

**2.** Vorrichtung (30) nach Anspruch 1, wobei der partielle Blutgasdruck ein partieller Kohlendioxiddruck ist, wobei der Blutgasdruckprozessor (38) als ein partieller Kohlendioxiddruckprozessor ausgelegt ist und wobei der Blutgasdruckprozessor (38) weiterhin konfiguriert ist, um pH-darstellende Werte zu ermitteln, die den vorliegenden Blut-pH-Werten der Person (12) zurechenbar sind.

**3.** Vorrichtung (30) nach Anspruch 1, wobei der Temperaturdetektor (32) weiterhin konfiguriert ist, um mindestens zwei Messzonen (52a, 52b, 52c) an der Haut der Person zu überwachen, wobei die mindestens zwei Messzonen (52a, 52b, 52c) unterschiedliche Bluttemperaturpegel aufweisen.

**4.** Vorrichtung (30) nach Anspruch 1, wobei der Temperaturdetektor (32) als ein optischer Detektor ausgelegt ist, der mindestens ein Sensorelement zum Erfassen von elektromagnetischer Strahlung umfasst, die tatsächliche Temperaturwerte angibt.

**5.** Vorrichtung (30) nach Anspruch 4, wobei der Temperaturdetektor (32) als ein Wärmebildgebungssensor ausgelegt ist, insbesondere als eine Wärmebildkamera.

**6.** Vorrichtung (30) nach Anspruch 4, wobei der Temperaturdetektor (32) weiterhin mindestens eine optische Temperaturanzeige (54) umfasst, die an der Haut der Person angebracht werden kann, wobei die mindestens eine optische Temperaturanzeige in der Lage ist, in Reaktion auf eine Temperaturänderung (54) eine Eigenschaftsänderung zu zeigen.

**7.** Vorrichtung (30) nach Anspruch 1, wobei der Sauerstoffsättigungssensor (34) als ein optischer Sensor ausgelegt ist, der in der Lage ist, elektromagnetische Strahlung mit mindestens zwei unterschiedlichen Wellenlängenabschnitten zu erfassen, die die Blutperfusion in einem Gewebe der Person angibt.

**8.** Vorrichtung (30) nach Anspruch 7, wobei der Sauerstoffsättigungssensor (34) als ein Bildsensor ausgelegt ist, insbesondere als eine Bildgebungskamera, die in der Lage ist, elektromagnetische Strahlung in mindestens einem bestimmten Wellenlängenbereich zu erfassen.

**9.** Vorrichtung (30) nach Anspruch 1, wobei der Temperaturdetektor (32) als ein Kontaktsensor ausgelegt ist, der an der Haut der Person angebracht werden kann.

**10.** Vorrichtung (30) nach Anspruch 1, wobei der Temperaturdetektor (32) und der Sauerstoffsättigungssensor (34) weiterhin in der Lage sind, mit Abtastraten zu arbeiten, die das Detektieren von Interzyklus-Schwankungen der Bluttemperatur und der Blutsauerstoffsättigung ermöglichen.

**11.** Vorrichtung (30) nach Anspruch 1, weiterhin umfassend einen Taktgenerator zum Synchronisieren des Temperaturdetektors (32) und des Sauerstoffsättigungssensors (34).

**12.** System zum Beatmen eines Patienten, wobei das System Folgendes umfasst:

- eine Vorrichtung (30) nach einem der vorhergehenden Ansprüche; und
- eine Patientenbeatmungsschnittstelle (14);

wobei der Temperaturdetektor (32) vorzugsweise an der genannten Patientenbeatmungsschnittstelle (14) angebracht ist.

**13.** Verfahren zur unaufdringlichen Bestimmung eines partiellen Blutgasdrucks in Blut in einem Kreislaufsystem einer Person von Interesse (12), wobei das Verfahren die folgenden Schritte umfasst:

- nicht-belästigendes Detektieren von temperaturbezogenen Messwerten, die die vorliegenden intrinsischen natürlichen Bluttemperaturpegeldifferenzen des Bluts einer Person angeben;
- nicht-belästigendes Ableiten von Sauerstoffsättigungsmessungen des Bluts der genannten Person (12) unter Berücksichtigung der genannten temperaturbezogenen Messwerte; und
- Ermitteln eines partiellen Blutgasdrucks der überwachten Person (12) anhand der genannten abgeleiteten Sauerstoffsättigungsmessungen unter Berücksichtigung der vorliegenden Bluttemperaturpegeldifferenzen der genannten Person (12)

**14.** Verfahren nach Anspruch 13, wobei der Schritt des Ermittelns des partiellen Blutgasdrucks Folgendes umfasst:

- Ermitteln von pH-darstellenden Werten, die den vorliegenden Blut-pH-Werten der Person (12) zurechenbar sind, vorzugsweise umfassend eine Ableitung der pH-darstellenden Werte von einer Sauerstoffdissoziationskurve unter Berücksichtigung von Änderungen in den genannten abgeleiteten Sauerstoffsättigungsmessungen des Bluts, die detektierten temperaturbezogenen Messwerten des Bluts der Person zuzurechnen sind.

**15.** Computerprogramm umfassend Programmcodemittel zum Veranlassen eines die Schritte des Verfahrens nach Anspruch 13 durchzuführen, wenn das genannte Computerprogramm auf dem Computer ausgeführt wird.


**Revendications**

**1.** Dispositif (30) pour déterminer de manière discrète une pression partielle de gaz sanguin dans le sang dans un système de circulation d'un sujet d'intérêt (12), le dispositif (30) comprenant :

- un détecteur de température discret (32) pour détecter des valeurs de mesure liées à la température indicatives de présentes différences de niveau de température de sang naturelle intrinsèques ;
- un capteur de saturation en oxygène discret (34) pour dériver des mesures de saturation en oxygène du sang dudit sujet (12) en prenant en considération lesdites valeurs de mesure liées à la température ; et
- un processeur de pression de gaz sanguin (38) pour déterminer une pression partielle de gaz sanguin du sujet contrôlé (12) à partir desdites mesures de saturation en oxygène dérivées en prenant en considération lesdites valeurs de mesure liées à la température indicatives des présentes différences de niveau de température de sang dudit sujet (12).

2. Dispositif (30) selon la revendication 1, dans lequel la pression partielle de gaz sanguin est une pression de dioxyde de carbone partielle, dans lequel le processeur de pression de gaz sanguin (38) est agencé sous la forme d'un processeur de pression de dioxyde de carbone partielle, et dans lequel le processeur de pression de gaz sanguin (38) est en outre configuré pour déterminer des valeurs représentatives du pH pouvant être attribuées à de présentes valeurs de pH de sang du sujet (12).

3. Dispositif (30) selon la revendication 1, dans lequel le détecteur de température (32) est en outre configuré pour contrôler au moins deux zones de mesure (52a, 52b, 52c) au niveau de la peau du sujet, dans lequel les au moins deux zones de mesure (52a, 52b, 52c) présentent des niveaux de température de sang différents.

4. Dispositif (30) selon la revendication 1, dans lequel le détecteur de température (32) est agencé sous la forme d'un détecteur optique comprenant au moins un élément sensoriel pour détecter un rayonnement électromagnétique indicatif de valeurs de température réelles.

5. Dispositif (30) selon la revendication 4, dans lequel le détecteur de température (32) est agencé sous la forme d'un capteur d'imagerie thermique, en particulier sous la forme d'une caméra d'imagerie thermique.

6. Dispositif (30) selon la revendication 4, dans lequel le détecteur de température (32) comprend en outre au moins un indicateur de température optique (54) pouvant être attaché à la peau du sujet, dans lequel l'au moins un indicateur de température optique est capable de présenter un changement de propriété en réponse à un changement de température (54).

7. Dispositif (30) selon la revendication 1, dans lequel le capteur de saturation en oxygène (34) est agencé sous la forme d'un capteur optique capable de détecter un rayonnement électromagnétique au niveau d'au moins deux portions de longueur d'onde distinctes indicatives de la perfusion sanguine dans un tissu du sujet.

8. Dispositif (30) selon la revendication 7, dans lequel le capteur de saturation en oxygène (34) est agencé sous la forme d'un capteur d'image, en particulier d'une caméra d'imagerie capable de détecter un rayonnement électromagnétique dans au moins une plage de longueur d'onde particulière.

9. Dispositif (30) selon la revendication 1, dans lequel le détecteur de température (32) est agencé sous la forme d'un capteur de contact pouvant être attaché à la peau du sujet.

10. Dispositif (30) selon la revendication 1, dans lequel le détecteur de température (32) et le capteur de saturation en oxygène (34) sont en outre capables de fonctionner à des vitesses d'échantillonnage permettant de détecter des variations inter-cycle de température de sang et de saturation en oxygène du sang.

11. Dispositif (30) selon la revendication 1, comprenant en outre un générateur d'horloge pour synchroniser le détecteur de température (32) et le capteur de saturation en oxygène (34).

12. Système pour ventiler un patient, le système comprenant

    - un dispositif (30) selon l'une quelconque des revendications précédentes ; et
    - une interface de ventilation de patient (14) ;

dans lequel le détecteur de température (32) est de préférence attaché à ladite interface de ventilation de patient (14).

13. Procédé pour déterminer de manière discrète une pression partielle de gaz sanguin dans le sang dans un système de circulation d'un sujet d'intérêt (12), comprenant les étapes suivantes :

    - la détection discrète de valeurs de mesure liées à la température indicatives de présentes différences de niveau de température naturelle intrinsèques du sang d'un sujet ;
    - la dérivation discrète de mesures de saturation en oxygène du sang dudit sujet (12) en prenant en considération lesdites valeurs de mesure liées à la température ; et

- la détermination d'une pression partielle de gaz sanguin du sujet contrôlé (12) à partir desdites mesures de saturation en oxygène dérivées en prenant en considération de présentes différences de niveau de température de sang dudit sujet (12).

**14.** Procédé selon la revendication 13, dans lequel l'étape de détermination de la pression partielle de gaz sanguin comprend :

- la détermination de valeurs représentatives du pH pouvant être attribuées à de présentes valeurs de pH de sang du sujet (12), de préférence comprenant une dérivation des valeurs représentatives du pH à partir d'une courbe de dissociation d'oxygène en prenant en considération des changements desdites mesures de saturation en oxygène dérivées du sang pouvant être attribuées à des valeurs de mesure liées à la température détectées du sang du sujet.

**15.** Programme informatique comprenant un moyen de code de programme pour amener un ordinateur qui fait partie d'un système ou dispositif médical à réaliser les étapes du procédé selon la revendication 13 quand ledit programme informatique est exécuté sur l'ordinateur.

FIG. 1

FIG. 2

FIG. 3

FIG. 4

FIG. 5

FIG. 6

FIG. 7

FIG. 8

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2485558 A
- WO 9803847 A2
- WO 2012077065 A1


**Non-patent literature cited in the description**

- **VERKRUYSSE et al.** Remote plethysmographic imaging using ambient light. *Optics Express,* 22 December 2008, vol. 16 (26), 21434-21445
- **WIERINGA et al.** Contactless Multiple Wavelength Photoplethysmographic Imaging: A First Step Toward "SpO2 Camera" Technology. *Ann. Biomed. Eng.,* 2005, vol. 33, 1034-1041